# EUROPEAN PATENT SPECIFICATION

(11) **EP 4 517 324 B1**
(45) Date of publication and mention of the grant of the patent: **31.12.2025**
(21) Application number: 24154659.7
(22) Date of filing: 30.01.2024
(51) Int. Cl.: G01N 33/543, G01N 33/76

(54) **TEST STRIP FOR DETECTING HUMAN CHORIONIC GONADOTROPIN IN SALIVA AND PREPARATION METHOD THEREOF**
TESTSTREIFEN ZUM NACHWEIS VON MENSCHLICHEM CHORIONGONADOTROPIN IN SPEICHEL UND HERSTELLUNGSVERFAHREN DAFÜR
BANDELETTE RÉACTIVE POUR DÉTECTER LA GONADOTROPHINE CHORIONIQUE HUMAINE DANS LA SALIVE ET SON PROCÉDÉ DE PRÉPARATION

(30) Priority: 28.08.2023 CN 202311091964
(43) Date of publication of application: 05.03.2025
(73) Proprietor: Bioteke Corporation (Wuxi) Co., Ltd., Wuxi, Jiangsu 214100 (CN)
(72) Inventor: LI, Pengcheng, Wuxi, Jiangsu, 214100 (CN); ZENG, Xiaoqiong, Wuxi, Jiangsu, 214100 (CN); ZHOU, Zhitu, Wuxi, Jiangsu, 214100 (CN)
(74) Representative: Schrell, Andreas

(56) References cited:
- WO-A2-2008/012650
- CN-A- 106 370 871
- CN-A- 112 326 641
- FR-A1- 3 115 110

## Description

### TECHNICAL FIELD

The present disclosure belongs to the technical field of test strip detection, and in particular relates to a test strip for detecting human chorionic gonadotropin in saliva and a preparation method thereof.

### BACKGROUND

Human chorionic gonadotropin (HCG) is a glycoprotein secreted by trophoblast cells of placenta and consists of α and β dimer glycoproteins, where α-subunit is common to anterior pituitary hormones, and β-subunit is specific to HCG. A main function of HCG is to stimulate corpus luteum, which is conducive to the continuous secretion of estrogen and progesterone, thereby promoting the formation of uterine decidua and making the placenta grow and mature. A modem view is that HCG is produced by transitional cells and syncytial cells of the trophoblast. HCG proliferates rapidly in the first 8 weeks of pregnancy to maintain the pregnancy, gradually decreases after about 8 weeks of the pregnancy, and reaches a relatively stable level until about 20 weeks of the pregnancy. HCG is an effective means of auxiliary diagnosis. The HCG content in the urine of pregnant women can be detected with HCG double antibodies and the results can be quickly obtained in the early stages of pregnancy.

Saliva, as a clinically informative biofluid, contains a variety of soluble biomarkers, enabling rapid testing or centralized clinical laboratory operations in a more standardized format. It is not uncommon for saliva to be used for *in vitro* testing, and saliva has been widely used to quantitatively detect steroid hormones such as cortisol, estriol, and testosterone. At present, there are already many rapid test strips for detecting HIV using saliva. Importantly, saliva is closely related to corresponding components in the blood. Saliva can be used as a window to blood, and relevant experimental results have shown that HCG concentration is well correlated with blood concentration. Therefore, saliva can serve as a non-invasive, rapid, and more acceptable biological fluid for pregnancy testing. A secretion mechanism of human chorionic villi in saliva may be that the saliva is a hypotonic liquid, and capillaries and capillary lymph nodes are closely connected to the basement membrane of glands; HCG can migrate from blood to saliva through the glandular intercellular space and tight junction leakage. Clinical tests related to HCG in saliva are conducted at the hospital, and both urine and saliva samples are tested simultaneously under the same conditions. The results show that pregnant women not only have HCG in their urine, but also in their saliva; and inspection results of the above two fluid samples can be compared, showing desirable correlation and consistency.

Saliva testing for early pregnancy is cleaner, more convenient, and not affected by the time and place of sample collection. However, test strips for detecting pregnancy in saliva are not generally mentioned, since there is a difficulty in development that saliva samples show many non-detection interferences, low sensitivity, difficult chromatography, and non-specificity. For example, the HCG content under normal circumstances is less than 5 mIU/mL, while women of pregnant age may be pregnant at an HCG content of greater than 5 mIU/mL. Most of the urine-based pregnancy test strips on the market have a limit of detection (LOD) of 25 mIU/mL and cannot detect pregnancy earlier. In the early stages of pregnancy, for example, within one week of pregnancy, the pregnancy cannot be detected out if the HCG content is not enough to reach 25 mIU/mL. In addition, it is difficult to avoid "polluting hands "regardless of the detection methods including urine dripping or urine cup dropper, and the urine dripping may also wet the detection window to affect chromatography results. Moreover, urine testing methods are also limited by time and location. Furthermore, when the sample of the test strip is saliva, since the saliva sample is relatively viscous, there is a poor flow rate of saliva when chromatographing upward from the sample pad; meanwhile, there are even problems such as latex microspheres blocking the nitrocellulose membrane, half development of the liquid during chromatography, and false positive results.
CN106370871A discloses a lateral flow test strip for testing HCG in saliva samples. FR3115110A1 discloses an immunological test kit for detecting virus infections, in particular SARS-CoV-2. CN11232664A discloses a method and a kit for quickly identifying sputum components from coughed samples. WO2008/012650A2 discloses an immunochromatography device for the diagnosis of diseases in a sample.

### SUMMARY

In view of this, an objective of the present disclosure is to provide a test strip for detecting HCG in saliva and a preparation method thereof. The test strip is clean and convenient for detecting early pregnancy, with high detection sensitivity and strong specificity.

To achieve the above objective, the present disclosure provides the following technical solutions:

The present disclosure provides a test strip for detecting HCG in saliva, including a sample pad, a conjugate pad, an absorbent pad, a coating membrane, and a substrate, where the sample pad, the conjugate pad, the absorbent pad, and the coating membrane are sequentially overlapped on the substrate;
the sample pad is a glass fiber membrane treated with a sample pad treatment solution; and
each 100 mL of the sample pad treatment solution includes the following components:
   1.0 g to 1.5 g of Tris, 0.3 g to 0.8g of casein, 50 µL to 100 µL of Tween-20, 0.5 g to 1.5 g of NaCl, 0.15 g to 0.2 g of dodecyltrimethylammonium bromide (DTAB), 0.8 g to 1.2 g of 4-nonylphenyl-polyethylene glycol, 3.5 g to 4.0 g of borax, 0.5 g to 1 g of polyvinylpyrrolidone (PVP), 3 g to 5 g of dithiothreitol (DTT), and 0.8 g to 2 g of L-cysteine.

Preferably, the sample pad treatment solution has a pH value of 8.0 to 8.5.

Preferably, the coating membrane is a nitrocellulose membrane coated with a test line (T line) and a control line (C line);
wherein the T line is sprayed with 0.5 mg/mL to 1 mg/mL of a mouse anti-α-HCG monoclonal antibody dilution;
the C line is sprayed with 0.4 mg/mL to 0.6 mg/mL of a goat anti-mouse immunoglobulin G (IgG) polyclonal antibody dilution; and
the T line or the C line is sprayed at an amount of 0.5 µL/cm to 1.5 µL/cm.

Preferably, the mouse anti-α-HCG monoclonal antibody dilution solution or the goat anti-mouse IgG polyclonal antibody dilution solution is prepared by coating the mouse anti-α-HCG monoclonal antibody or the goat anti-mouse IgG polyclonal antibody with a coating diluent; and the coating diluent is a PBS solution with 50 mg/mL to 150 mg/mL of trehalose.

Preferably, the conjugate pad is a glass fiber membrane or a polyester fiber membrane treated with a conjugate pad treatment solution; and
the conjugate pad treatment solution includes the following components: 1 g to 1.5 g of 3-(N-morpholino)-2-hydroxy-propanesulfonic acid, 0.2 g to 1 g of casein, 50 µL to 100 µL of Tween-20, and 75 mL to 85 mL of water.

Preferably, a β-HCG latex microsphere is sprayed on the glass fiber membrane or the polyester fiber membrane at 1.5 µL/cm to 3 µL/cm.
wherein the β-HCG latex microsphere is prepared by the following steps:
washing a red latex microsphere with a borax buffer to obtain a washed red latex microsphere;
mixing an anti-β-HCG antibody (a labeling antibody) with the washed red latex microsphere, and activating a resulting mixture using 1-ethyl-3-(3-dimethylaminopropyl)carbodiimide hydrochloride (EDC·HCl) solution to obtain an anti-β-HCG antibody-modified red latex microsphere; and
mixing an ethanolamine solution with the anti-β-HCG antibody-modified red latex microsphere to allow blocking to obtain a blocked β-HCG red latex microsphere, conducting centrifugation and removing a resulting supernatant; ultrasonically mixing the blocked β-HCG red latex microsphere with a Tris buffer evenly, conducting centrifugation and removing a resulting supernatant; ultrasonically mixing a remaining product with the Tris buffer evenly, and conducting rotary blending at a room temperature for 4 h to 6 h to obtain the β-HCG latex microsphere.

The present disclosure further provides a preparation method of the test strip, including the following steps:
(1) preparation of the sample pad:
   allowing the glass fiber membrane to absorb the sample pad treatment solution evenly, and drying the glass fiber membrane to obtain the sample pad; wherein the sample pad treatment solution comprises the following components in each 100 mL of sample pad treatment solution:
   1.0 g to 1.5 g of Tris, 0.3 g to 0.8g of casein, 50 µL to 100 µL of Tween-20, 0.5 g to 1.5 g of NaCl, 0.15 g to 0.2 g of dodecyltrimethylammonium bromide (DTAB), 0.8 g to 1.2 g of 4-nonylphenyl-polyethylene glycol, 3.5 g to 4.0 g of borax, 0.5 g to 1 g of polyvinylpyrrolidone (PVP), 3 g to 5 g of dithiothreitol (DTT), and 0.8 g to 2 g of L-cysteine;
(2) preparation of the conjugate pad:
   allowing the glass fiber membrane or the polyester fiber membrane to absorb the conjugate pad treatment solution evenly, drying the glass fiber membrane or the polyester fiber membrane, and spraying the β-HCG latex microsphere on the glass fiber membrane or the polyester fiber membrane treated with the conjugate pad treatment solution at a spraying volume of 1.5 µL/cm to 3 µL/cm to obtain the conjugate pad;
   wherein the β-HCG latex microsphere is prepared by the following steps;
   washing a red latex microsphere with a borax buffer to obtain a washed red latex microsphere;
   mixing an anti-β-HCG antibody with the washed red latex microsphere, and activating a resulting mixture using 1-ethyl-3-(3-dimethylaminopropyl)carbodiimide hydrochloride (EDC·HCl) solution to obtain an anti-β-HCG antibody-modified red latex microsphere; and
   mixing an ethanolamine solution with the anti-β-HCG antibody-modified red latex microsphere to allow blocking to obtain a blocked β-HCG red latex microsphere, conducting centrifugation and removing a resulting supernatant; ultrasonically mixing the blocked β-HCG red latex microsphere with a Tris buffer evenly, conducting centrifugation and removing a resulting supernatant; ultrasonically mixing a remaining product with the Tris buffer evenly, and conducting rotary blending at a room temperature for 4 h to 6 h to obtain the β-HCG latex microsphere;
(3) preparation of the coating membrane:
   diluting a mouse anti-α-HCG monoclonal antibody and a goat anti-mouse IgG polyclonal antibody with the coating diluent to obtain the mouse anti-α-HCG monoclonal antibody dilution and the goat anti-mouse IgG polyclonal antibody dilution, respectively; spraying the mouse anti-α-HCG monoclonal antibody dilution on the T line of the nitrocellulose membrane, spraying the goat anti-mouse IgG polyclonal antibody dilution on the C line of the nitrocellulose membrane, and drying the nitrocellulose membrane to obtain the coating membrane; and
(4) preparation of the test strip:
   cutting the sample pad, the conjugate pad, and the absorbent pad separately, and pasting, layer by layer, the sample pad, the conjugate pad, the coating membrane, and the absorbent pad on the substrate in sequence; where one end of the conjugate pad is arranged above the sample pad, the other end of the conjugate pad is arranged below one end of the coating membrane, the other end of the coating membrane is arranged below the absorbent pad, and the C line on the coating membrane is close to the absorbent pad.

Preferably, a preparation process of the β-HCG latex microsphere includes the following steps:
washing a red latex microsphere with a borax buffer to obtain a washed red latex microsphere;
mixing an anti-β-HCG antibody with the washed red latex microsphere, and activating a resulting mixture using 1-ethyl-3-(3-dimethylaminopropyl)carbodiimide hydrochloride (EDC·HCl) solution to obtain an anti-β-HCG antibody-modified red latex microsphere; and
mixing an ethanolamine solution with the anti-β-HCG antibody-modified red latex microsphere to allow blocking to obtain a blocked β-HCG red latex microsphere, conducting centrifugation and removing a resulting supernatant; ultrasonically mixing the blocked β-HCG red latex microsphere with a Tris buffer evenly, conducting centrifugation and removing a resulting supernatant; ultrasonically mixing a remaining product with the Tris buffer evenly, and conducting rotary blending at a room temperature for 4 h to 6 h to obtain the β-HCG latex microsphere.

Preferably, the anti-β-HCG antibody, the washed red latex microsphere, and the EDC·HCl solution are added at 1 mg: 10 mg: 0.05 mL.

The present disclosure further provides a kit for detecting early pregnancy, including the test strip and a sample diluent.

Compared with the prior art, the present disclosure has the following beneficial effects:
The present disclosure provides a test strip for detecting HCG in saliva and a preparation method thereof. In the present disclosure, saliva is collected directly and a sample pad is treated with a sample pad treatment solution, such that the saliva sample increases its water solubility, making the saliva sample smoothly chromatographedto reduce the interference of other impurity components in saliva, thereby eliminating a false positive phenomenon in saliva sample detection and completely eliminating non-specific reactions. At the same time, the test strip has desirable stability and can be stored at room temperature for a long time. The test strip has a LOD of 5 mIU/mL and can detect pregnancy earlier. The test strip can serve as a potential, user-friendly, and highly-acceptable product that is cleaner, more convenient, and independent of the time and place of sample collection.

### BRIEF DESCRIPTION OF THE DRAWINGS

FIG. 1 shows a schematic diagram of determining whether there is pregnancy detected by the test strip of the present disclosure;
FIG. 2 shows specificity test results of the test strips for detecting HCG in saliva using different test cards; where there are the test results using the test cards of Comparative Example 3, Comparative Example 2, Comparative Example 1, and Example 2 from left to right;
FIG. 3 shows specificity test results of the test strips for detecting HCG in saliva using different test cards after being stored at room temperature for 182 d; where there are the test results using the test card of Comparative Example 2, Comparative Example 1, and Example 2 from left to right;
FIG. 4 shows test results of 20 negative saliva samples; and
FIG. 5 shows test results of 8 positive saliva samples.

### DETAILED DESCRIPTION OF THE EMBODIMENTS

The present disclosure provides a test strip for detecting HCG in saliva, including a sample pad, a conjugate pad, an absorbent pad, a coating membrane, and a substrate, where the sample pad, the conjugate pad, the absorbent pad, and the coating membrane are sequentially overlapped on the substrate;
the sample pad is a glass fiber membrane treated with a sample pad treatment solution; and
each 100 mL of the sample pad treatment solution includes the following components:
   1.0 g to 1.5 g of Tris, 0.3 g to 0.8g of casein, 50 µL to 100 µL of Tween-20, 0.5 g to 1.5 g of NaCl, 0.15 g to 0.2 g of dodecyltrimethylammonium bromide (DTAB), 0.8 g to 1.2 g of 4-nonylphenyl-polyethylene glycol, 3.5 g to 4.0 g of borax, 0.5 g to 1 g of polyvinylpyrrolidone (PVP), 3 g to 5 g of dithiothreitol (DTT), and 0.8 g to 2 g of L-cysteine.

In the present disclosure, the sample pad has a width of preferably 2 cm±0.1 cm and a length of preferably 30 cm±0.2 cm; the absorbent pad is available from Whatman with a catalog number of 8151-6621.

In the present disclosure, the sample pad treatment solution further includes preferably 750 mL to 850 mL of water, and the sample pad treatment solution has a pH value of preferably 8.0 to 8.5. A preparation process of the sample pad treatment solution preferably includes: sequentially weighing 1.0 g to 1.5 g of Tris, 0.3 g to 0.8 g of casein, 50 µL to 100 µL of Tween-20, 0.5 g to 1.5 g of NaCl, 0.15 g to 0.2 g of DTAB, 0.8 g to 1.2 g of 4-nonylphenyl-polyethylene glycol, 3.5 g to 4.0 g of borax, 0.5 g to 1 g of PVP, adding 750 mL to 850 mL of water, dissolving a resulting mixture by stirring; adding 3 g to 5 g of DTT and 0.8 g to 2 g of L-cysteine, dissolving a resulting mixture by stirring; and adjusting a resulting mixture to a pH value of 8.0 to 8.5 and diluting to 100 mL to obtain the sample pad treatment solution. A combination of DTT and L-cysteine in the sample pad treatment solution can eliminate non-specific reactions during the chromatography process; meanwhile, DTT has an antioxidant effect and can prevent L-cysteine from being oxidized into cystine by air to form a precipitate and has a synergistic effect. DTT and L-cysteine are generally formulated into a solution for future use. The solution is easily oxidized and then not conducive to storage due to a short validity period and usually needs to be used as soon as possible or stored at a low temperature. Frozen storage conditions are obviously impractical for products such as rapid testing reagents. Therefore, the above two reagents and other components of the sample pad treatment solution are placed in the sample pad, thus effectively solving the problem of short validity period of the reagents; the reagents can be stored at room temperature for a long time, showing a validity period of not less than 6 months. 4-nonylphenyl-polyethylene glycol is a wetting agent P-40 purchased from Jiangsu Pules Bio-tech Co., Ltd.

In the present disclosure, the test strip adopts a new sample pad processing method, such that saliva drips into the sample pad and reacts with the active ingredients in the sample pad. Due to the reduced viscosity of saliva, the chromatography is extremely smooth without the occurrence of film clogging during operations. This measure increases specificity of the test strip, reduces an interference of other components in saliva and avoids a possibility of false positive results in saliva samples.

In the present disclosure, the coating membrane is preferably a nitrocellulose membrane coated with a T line and a C line; the T line is preferably sprayed with 0.5 mg/mL to 1 mg/mL of a mouse anti-α-HCG monoclonal antibody dilution; the C line is preferably sprayed with 0.4 mg/mL to 0.6 mg/mL of a goat anti-mouse IgG polyclonal antibody dilution; and the T-line or the C-line is sprayed at preferably 0.5 µL/cm to 1.5 µL/cm. The mouse anti-α-HCG monoclonal antibody dilution or the goat anti-mouse IgG polyclonal antibody dilution is prepared by a coating diluent; and the coating diluent is a PBS solution with 50 mg/mL to 150 mg/mL of trehalose. The PBS solution preferably has a concentration of 0.005 M to 0.015 M. The T line and the C line on the coating membrane have a spacing distance of preferably 0.4 cm to 0.6 cm. The selected antibody raw materials increase sensitivity while reducing the occurrence of non-specific reactions. The mouse anti-α-HCG monoclonal antibody is purchased from Nanjing Genstars Biotech Co., Ltd. and has a product number of W4003; the mouse anti-α-HCG monoclonal antibody is purchased from Artron BioResearch (Shandong) Co., Ltd. and has a product number of A01-Ab2.

In the present disclosure, the conjugate pad is preferably a glass fiber membrane or a polyester fiber membrane treated with a conjugate pad treatment solution; and
the conjugate pad treatment solution includes preferably the following components: 1 g to 1.5 g of 3-(N-morpholino)-2-hydroxy-propanesulfonic acid, 0.2 g to 1 g of casein, 50 µL to 100 µL of Tween-20, and 75 mL to 85 mL of water. The conjugate pad treatment solution has a pH value of preferably 8.0 to 8.5. A preparation process of the conjugate pad treatment solution includes: weighing 1 g to 1.5 g of 3-(N-morpholino)-2-hydroxy-propanesulfonic acid, 0.2 g to 1 g of casein, 50 µL to 100 µL of Tween-20, and 75 mL to 85 mL of water, dissolving by stirring, adjusting a resulting mixture to a pH value of 8.0 to 8.5, and diluting to 100 mL. As a preferred example, the glass fiber membrane or the polyester fiber membrane is sprayed with a β-HCG latex microsphere at a spraying volume of 1.5 µL/cm to 3 µL/cm. The 3-(N-morpholino)-2-hydroxy-propanesulfonic acid is purchased from Damas-beta, with a catalog number of 76496B.

In the present disclosure, the conjugate pad has a width of preferably 1 cm±0.2 cm and a length of preferably 30 cm±0.2 cm.

The present disclosure further provides a preparation method of the test strip, including the following steps:
(1) preparation of the sample pad:
   allowing the glass fiber membrane to absorb the sample pad treatment solution evenly, and drying the glass fiber membrane to obtain the sample pad;
(2) preparation of the conjugate pad:
   allowing the glass fiber membrane or the polyester fiber membrane to absorb the conjugate pad treatment solution evenly, drying the glass fiber membrane or the polyester fiber membrane, and spraying the β-HCG latex microsphere on the glass fiber membrane or the polyester fiber membrane treated with the conjugate pad treatment solution at a spraying volume of 1.5 µL/cm to 3 µL/cm to obtain the conjugate pad;
(3) preparation of the coating membrane:
   diluting a mouse anti-α-HCG monoclonal antibody and a goat anti-mouse IgG polyclonal antibody with the coating diluent to obtain the mouse anti-α-HCG monoclonal antibody dilution and the goat anti-mouse IgG polyclonal antibody dilution, respectively; spraying the mouse anti-α-HCG monoclonal antibody dilution on the T line of the nitrocellulose membrane, spraying the goat anti-mouse IgG polyclonal antibody dilution on the C line of the nitrocellulose membrane, and drying the nitrocellulose membrane to obtain the coating membrane; and
(4) preparation of the test strip:
   cutting the sample pad, the conjugate pad, and the absorbent pad separately, and pasting the sample pad, the conjugate pad, the coating membrane, and the absorbent pad on the substrate in sequence; where one end of the conjugate pad is arranged above the sample pad, the other end of the conjugate pad is arranged below one end of the coating membrane, the other end of the coating membrane is arranged below the absorbent pad, and the C line on the coating membrane is close to the absorbent pad.

In the present disclosure, the preparation of the sample pad: the glass fiber membrane is made by absorbing the sample pad treatment solution evenly, and dried to obtain the sample pad. The drying is preferably conducted at 40°C to 45°C for not less than 2 h.

In the present disclosure, the glass fiber membrane or the polyester fiber membrane absorbs the conjugate pad treatment solution evenly and then is dried. The drying is preferably conducted at 40°C to 45°C for 8 h to 12 h.

In the present disclosure, the β-HCG latex microsphere is sprayed on the glass fiber membrane or the polyester fiber membrane treated with the conjugate pad treatment solution at a spraying volume of 1.5 µL/cm to 3 µL/cm to obtain the conjugate pad. A preparation process of the β-HCG latex microsphere includes the following steps: washing a red latex microsphere with a borax buffer to obtain a washed red latex microsphere; mixing an anti-β-HCG antibody with the washed red latex microsphere, and activating a resulting mixture using a 1-ethyl-3-(3-dimethylaminopropyl)carbodiimide hydrochloride (EDC·HCl) solution to obtain an anti-β-HCG antibody-modified red latex microsphere; and mixing an ethanolamine solution with the anti-β-HCG antibody-modified red latex microsphere to allow blocking to obtain a blocked β-HCG red latex microsphere, conducting centrifugation and removing a resulting supernatant; ultrasonically mixing the blocked β-HCG red latex microsphere with a Tris buffer evenly, conducting centrifugation and removing a resulting supernatant; ultrasonically mixing a remaining product with the Tris buffer evenly, and conducting rotary blending at a room temperature for 4 h to 6 h to obtain the β-HCG latex microsphere.

In the present disclosure, a red latex microsphere is washed with a borax buffer to obtain a washed red latex microsphere. The red latex microsphere has a particle size of preferably 150 nm to 250 nm, more preferably 200 nm. The red latex microsphere and the borax buffer are at a volume ratio of preferably (0.1-0.2):1. The borax buffer has a concentration of preferably 0.05 M to 0.15 M. The washing preferably includes: centrifuging at 13,500 rpm for 10 min to 20 min, removing a resulting supernatant, and then adding 0.5 mL to 1.5 mL of the borax buffer to the washed red latex microsphere after centrifugation to allow ultrasonic mixing. The red latex microsphere is purchased from Suzhou VDO Biotech Co., Ltd., and has a product number of DR0200CA-1.

In the present disclosure, an anti-β-HCG antibody is mixed with the washed red latex microsphere, and a resulting mixture is activated using an EDC·HCl solution to obtain an anti-β-HCG antibody-modified red latex microsphere. The anti-β-HCG antibody, the washed red latex microsphere, and the EDC·HCl solution are added at 1 mg: 10 mg: 0.05 mL. The EDC·HCl solution is preferably an EDC·HCl solution of 5 mg/mL to 15 mg/mL. A preparation process of the 5 mg/mL to 15 mg/mL EDC·HCl solution preferably includes: dissolving 5 mg to 15 mg of the EDC·HCl solution in 1 mL of purified water. The activating is preferably conducted by shaking and mixing well at a room temperature for 4 h to 8 h. The anti-β-HCG antibody is purchased from Chuangsheng Biotech (Shandong) Co., Ltd., with a product number of A01-Ab1.

In the present disclosure, an ethanolamine solution is mixed with the anti-β-HCG antibody-modified red latex microsphere to allow blocking to obtain a blocked β-HCG red latex microsphere. The ethanolamine solution is preferably an ethanolamine solution with a concentration of 0.08 M to 0.12 M. The blocking is conducted for preferably 15 min to 30 min.

In the present disclosure, the Tris buffer and the blocked β-HCG red latex microsphere are ultrasonically mixed and then subjected to rotary blending at a room temperature for 4 h to 6 h. The Tris buffer is preferably a Tris buffer with a concentration of 50 mM to 100 mM.

In the present disclosure, the β-HCG latex microsphere is obtained by adding the Tris buffer and conducting ultrasonic homogenization. The Tris buffer has a volume of preferably 0.4 mL to 0.8 mL, and the ultrasonic mixing is conducted for preferably 10 min to 20 min.

In the present disclosure, after spraying the β-HCG latex microsphere, drying is conducted at 37°C to 55°C for 20 min to 60 min.

In the present disclosure, the test strip is labeled with the latex microsphere instead of colloidal gold, and the red latex microsphere with a particle size of 200 nm is selected to eliminate an influence of the viscosity of some saliva samples.

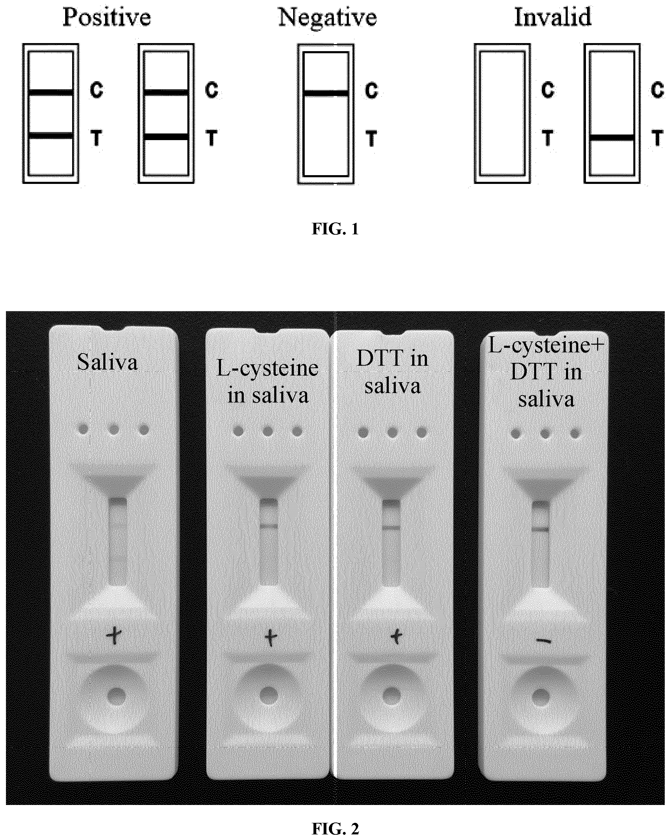

In the present disclosure, the test strip is used to detect and determine whether pregnancy is achieved according to the following criteria (FIG. 1):
(1) Positive (pregnancy): there are two red reaction lines, one in the test region (T) and one in the control region (C); if the color of the test region (T) is extremely weak, it means early pregnancy and needs to be retested the next day.
(2) Negative (no pregnancy): a red reaction line, that is, only a red reaction line appears in the control region (C).
(3) Invalid: no red reaction line appears in the control region (C), indicating that the test is wrong or invalid.

When the HCG content in pregnant women is greater than 5 mIU/mL, there is a possibility of pregnancy; in the early stages of pregnancy, for example, within 10 d, when the HCG content is greater than 5 mIU/mL but less than 25 mIU/mL, the pregnancy cannot be detected by commonly used test strips with lower sensitivity. In the present disclosure, the test strip has a minimum LOD of 5 mIU/mL and can detect pregnancy earlier. The minimum LOD refers to a lowest concentration that is greater than or equal to 95% detection rate.

The present disclosure further provides a kit for detecting early pregnancy, including the test strip and a sample diluent.

The sample diluent is preferably 0.9% physiological saline, and the sample diluent increases a liquefaction effect of the saliva sample. The saliva sample and the sample diluent during detection are added at a volume ratio of 1:1. The kit further includes a small funnel, which is used for collecting saliva. A cap of a sample treatment tube is opened, the matching saliva funnel is placed at a mouth of the tube, the saliva is added into the saliva funnel, the saliva funnel is taken out, the cap of the sample treatment tube is covered, and the sample and sample diluent are mixed well and dripped to allow testing. The kit can be read within 5 min, and is hygienic, convenient, and not affected by the time and place of sample collection.

The technical solution provided by the present disclosure will be described in detail below with reference to the examples, but they should not be construed as limiting the claimed scope of the present invention.

### Example 1

A preparation method of a test strip for detecting HCG in saliva included the following steps:

### (1) preparation of the sample pad:

Preparation of a sample pad treatment solution: 800 mL of purified water was added in a 100 mL small beaker, 1.21 g of Tris, 0.5 g of casein, 80 µL of Tween-20, 0.9 g of NaCl, 0.15 g of DTAB, 1.0 g of 4-nonylphenyl-polyethylene glycol, 3.811 g of borax, and 0.8 g of PVP were added into the small beaker in sequence, dissolved by stirring one by one; after all the above materials were evenly dissolved, 3 g of DTT and 1.2 g of L-cysteine were added, dissolved by stirring, a resulting mixture was adjusted to a pH value of 8.5, and diluted to 100 mL for later use.

A sample pad treatment solution was prepared according to the formula, poured on a glass fiber sheet, the glass fiber was gently scraped with tweezers to guide the treatment solution to be evenly absorbed by the glass fiber membrane; after the treatment solution was evenly absorbed by glass fiber membrane, two diagonal corners of the glass fiber membrane were clamped with tweezers and quickly transferred to a water draining grid; the water draining grid was placed in an oven to dry at 45°C for 4 h until excess water evaporated, and a relative humidity of the oven was less than or equal to 30%.

### (2) preparation of the conjugate pad:

### S1. Washing red latex microsphere

0.1 mL of 200 nm red latex microsphere was added into a 2 mL centrifuge tube containing 1 mL of 0.1 M borax buffer, and mixed thoroughly. A resulting mixture was centrifuged at 14,000 rpm for 15 min, a resulting supernatant was removed by pipetting, the red latex microsphere was washed again and added with 1 mL of 0.1 M borax buffer, and the latex microsphere in the centrifuge tube was ultrasonically mixed using an ultrasonic cell disrupter.

### S2. Labeling washed red latex microsphere with anti-β-HCG antibody

0.5 mg of anti-β-HCG antibody at a final concentration of 1 mg/mL was pipetted into 1 mL of the washed red latex microsphere, and then added with 25 µL of a latex microsphere activator (8 mg/mL of an EDC·HCl salt solution), mixed well by shaking for 1 min, and blended evenly with a shaker or rotator at room temperature for 5 h to obtain an anti-β-HCG antibody-modified red latex microsphere.

### S3. Blocking

50 µL of a blocking solution (0.1 M ethanolamine solution) was added to approximately 1 mL of the anti-β-HCG antibody-modified red latex microsphere, and mixed for 15 min to 30 min; an obtained mixture was centrifuged to remove a supernatant, 1 mL of a preservation solution (100 mM Tris buffer) was added to a blocked β-HCG red latex microsphere, ultrasonically mixed for 4 h to 6 h, and centrifuged to remove a supernatant; the preservation solution with a final volume of 0.5 mL was added, and sonicated in a water bath for 15 min to obtain a β-HCG latex microsphere.

### S4. Spraying the β-HCG latex microsphere

Preparation of conjugate pad treatment solution: 1.13 g of 3-(N-morpholino)-2-hydroxy-propanesulfonic acid, 0.5 g of casein, and 50 µL of Tween-20 were added into a small beaker filled with 80 mL of purified water, dissolved by stirring; a resulting mixture was adjusted to a pH value of 8.5, and diluted to 100 mL for later use.

Treatment of conjugate pad: the prepared conjugate pad treatment solution was poured on a piece of polyester fiber membrane, the polyester fiber membrane was gently scraped with tweezers to guide the treatment solution to be evenly absorbed by the polyester fiber membrane; after the treatment solution was evenly absorbed by polyester fiber membrane, two diagonal corners of the polyester fiber membrane were clamped with tweezers and quickly transferred to a water draining grid;
the water draining grid was placed in an oven and dried at 45°C for 8 h until excess water evaporated, and relative humidity of the oven was less than or equal to 30%.

20 mg of trehalose was added into a 1.5 mL centrifuge tube, in a weighing range of ±0.001 g of a calculated amount. 80 µL of latex diluent and 20 µL of β-HCG latex microsphere were added to a 1.5 mL centrifuge tube containing 20 mg of trehalose and shaken well, where the latex diluent was a Tris-Casein buffer prepared from 100 mM Tris buffer and 2.5 mM Casein. The treated conjugate pad was sprayed with the β-HCG latex microsphere using a gold-spraying slider, with a spray volume of 3 µL/cm, and the conjugate pad after spraying the β-HCG latex microsphere was dried in a 55°C oven with a relative humidity of less than or equal to 30% for 20 min.

### (3) preparation of the coating membrane:

An antibody coating diluent was used: a coating diluent was 0.01 M PBS and trehalose with a final concentration of 100 mg/mL; a mouse anti-α-HCG monoclonal antibody and a goat anti-mouse IgG polyclonal antibody were diluted to obtain a goat anti-mouse IgG polyclonal antibody dilution (C line coating solution) 0.5 mg/mL and a mouse anti-α-HCG monoclonal antibody dilution (T line coating solution) 0.5 mg/mL, respectively; the coating diluent was streaked on a substrate with the nitrocellulose membrane using a spraying and streaking device at a spray volume of 1 µL/cm, to obtain uniform and continuous C and T lines parallelly arranged on the membrane; and the coating membrane was dried in an oven.

### (4) preparation of the test strip:

### S1. Assembly of test strip:

1) Cutting strips: the sample pad was cut to a width of 2 cm±0.1 cm and a length of 30 cm±0.2 cm; the HCG latex conjugate pad was cut to a width 1 cm±0.2 cm and a length of 30 cm±0.2 cm; and the absorbent pad was cut to a length of 30 cm±0.1 cm and a width of 4 mm.
2) The substrate was placed flat on a table, and one end of the absorbent pad was attached on the nitrocellulose membrane to contact with a lower edge of the nitrocellulose membrane by 2 mm.
3) One end of the conjugate pad sprayed with the β-HCG latex microsphere (also called latex conjugate pad) on the other end of the nitrocellulose membrane, to contact with a lower edge of the latex conjugate pad by 2 mm.
4) One end of the sample pad was placed under the other end of the latex conjugate pad, to contact with the lower edge of the latex conjugate pad by 2 mm.
5) Paste a connecting tape: The connecting tape covered a connection point of the sample pad-conjugate pad-absorbent pad, and connected the upper edge of the absorbent pad to the lower edge of the nitrocellulose membrane, and the tape was pressed using a smooth side of the peeled-off sticker along one side.
6) An assembled large panel entered strip cutting processes.

### S2. Strip cutting (in a clean area with relative humidity less than or equal to 30%)

A cutting machine was started, a cutting program was set, and a cutting width was set to 3.05 mm; the large panel was slowly placed on a film cutting machine end to end. The large panel was placed flatly into a platform track of the film cutting machine, with its front side facing up, screws on the cutting machine were adjusted to make a width of the card slot to be the same as the width of the large panel, and then the strip cutting was started; each component of the test strip should be intact without being damaged or falling off to obtain a final product of the test strip.

### Example 2

A preparation method of a test card for detecting HCG in saliva included: the test strip prepared in Example 1 was assembled into a card (in a clean area with a relative humidity less than or equal to 30%). That is, each test strip was assembled into a card case and the upper cover was covered; the test card was assembled and passed the quality test to obtain final products.

### Example 3

A kit for detecting early pregnancy included the test card prepared in Example 2, a small funnel, and a sample diluent (0.9% physiological saline).

### Example 4

A detection method using the kit of Example 3 included the following steps:

### 1. Collection of samples

The subject's mouth was kept clean 10 min before the test, and food intake (including drinks, coffee, and food), smoking, or oral sprays were limited.

The cap of the sample treatment tube was opened, the matching saliva funnel was placed at the mouth of the tube, the saliva from the mouth was added dropwise into the saliva funnel, and the liquid level in the sample treatment tube was observed, where a saliva volume should be as consistent as possible with that of the sample diluent.

### 2. Sample treatment

The saliva funnel was removed, the cap of sample treatment tube was covered, and the sample was mixed as well as possible by repeatedly inverting the tube upside down for at least 10 seconds.

### (3) Test

The test card was taken out by tearing along the incision of the packaging aluminum bag and placed flat on the table, and 3 drops of the processed sample was added into a sampling hole of the test card. After starting timing, the results should be determined within 5 min to 10 min, and the results after 15 min were regarded invalid.

### 4. Interpretation of test results

(1) Positive (pregnancy): there are two red reaction lines, one in the test region (T) and the other in the control region (C); if the color of the test region (T) is extremely weak, it means early pregnancy and needs to be retested the next day.
(2) Negative (no pregnancy): a red reaction line, that is, only a red reaction line appears in the control region (C).
(3) Invalid: no red reaction line appears in the control region (C), indicating that the test is wrong or invalid.

### Example 5

### Sensitivity test of the test strip for detecting HCG in saliva

An HCG national standard was prepared into 4 concentrations: 2.5 mIU/mL, 5 mIU/mL, 10 mIU/mL, and 20 mIU/mL. The above four concentrations of HCG were detected using the detection method of Example 4, and the test was repeated 10 times. The results are shown in Table 1.

**Table 1 Sensitivity test results of test strip**

| Number of repetitions | 2.5 mIU/mL | 5 mIU/mL | 10 mIU/mL | 20 mIU/mL |
|---|---|---|---|---|
| 1 | + | + | + | + |
| 2 | + | + | + | + |
| 3 | + | + | + | + |
| 4 | - | + | + | + |
| 5 | + | + | + | + |
| 6 | + | + | + | + |
| 7 | - | + | + | + |
| 8 | + | + | + | + |
| 9 | + | + | + | + |
| 10 | - | + | + | + |
| Detected positive results | 7 | 10 | 10 | 10 |
| Positive rate | 70% | 100% | 100% | 100% |

| | | | | |
|---|---|---|---|---|
| "+" in Table 1 indicated detected, and "-" indicated non-detected. | | | | |

The results in Table 1 show that the detection rate at concentrations of 5 mIU/mL to 20 mIU/mL was 100%, and the detection rate at 2.5 mIU/mL was 70%. Therefore, this product had high sensitivity with an LOD of 5 mIU/mL.

### Comparative Example 1

This comparative example differed from Example 2 in that: 3 g of DTT was not added when preparing the sample pad treatment solution, while the remaining steps were the same as those in Example 2.

### Comparative Example 2

This comparative example differed from Example 2 in that: 1.2 g of L-cysteine was not added when preparing the sample pad treatment solution, while the remaining steps were the same as those in Example 2.

### Comparative Example 3

This comparative example differed from Example 2 in that: 3 g of DTT and 1.2 g of the L-cysteine were not added when preparing the sample pad treatment solution, while the remaining steps were the same as those in Example 2.

### Example 6

### Specificity test of the test strip for detecting HCG in saliva

The test cards prepared in Example 2 and Comparative Examples 1 to 3 were tested on the same saliva sample with reference to the detection method in Example 4. The results are shown in FIG. 2 and Table 2.

**Table 2 Test results for eliminating non-specific reactions**

| Group | Comparative Example 3 | Comparative Example 2 | Comparative Example 1 | Example 2 |
|---|---|---|---|---|
| Results | +++ | + | + | - |

| | | | | |
|---|---|---|---|---|
| Note: "+++" denotes clearly visible, "++" denotes visible, "+" denotes weakly visible, and "-" denotes invisible. | | | | |

The results are shown in FIG. 2 and Table 2. When L-cysteine or DTT were used alone, the specificity problems caused by the viscosity of saliva samples were improved, but they did not completely eliminate non-specific reactions. Moreover, the test region of the test strip showed problems such as a red background and obvious microsphere residues. The two reagents L-cysteine and DTT were used together with satisfactory results. From the comparison in FIG. 2, it is seen that the combination of the two reagents L-cysteine and DTT completely eliminates the non-specific reactions, with a background obviously clean.

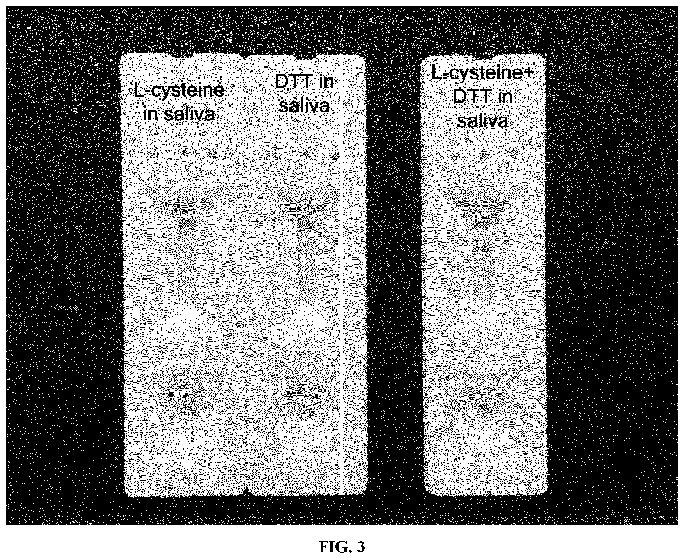

Further, the test cards prepared in Example 2 and Comparative Examples 1 to 3 were stored at room temperature for 182 d and then tested on the same saliva sample according to the detection method of Example 4. The results are shown in FIG. 3 and Table 3.

**Table 3 Tests after 182 days of storage at room temperature**

| Group | Comparative Example 2 | Comparative Example 1 | Example 2 |
|---|---|---|---|
| Results | ++ | ++ | - |

| | | | |
|---|---|---|---|
| Note: "+++" denotes clearly visible, "++" denotes visible, "+" denotes weakly visible, and "-" denotes invisible. | | | |

As shown in FIG. 2 and Table 3, the effect of eliminating specific reactions of L-cysteine and DTT is reduced when used alone, but their combination could still completely eliminate the specific reactions.

In addition, the test cards prepared in Example 2 and Comparative Example 3 were used to test 120 negative saliva samples with reference to the detection method of Example 4. The results are shown in Table 4.

**Table 4 Test results of 120 negative saliva samples with different test cards**

| Group | Result (number of negative cases) | Number of matching cases | Number of non-matching cases | Matching ratio |
|---|---|---|---|---|
| Comparative Example 3 | 13 | 13 | 107 | 10.8% |
| Example 2 | 120 | 120 | 0 | 100% |

As shown in Table 4, the sample pad treatment method of the test strip in the present disclosure can effectively eliminate non-specific reactions, with an excellent negative coincidence rate.

### Example 7

### Real sample test using the test strip for detecting HCG in saliva

The saliva samples of 20 non-pregnant and 8 women diagnosed as pregnant were collected and tested using the test card of Example 2 with reference to the detection method of Example 4, and the chromatography results of the test card were observed after 5 min.

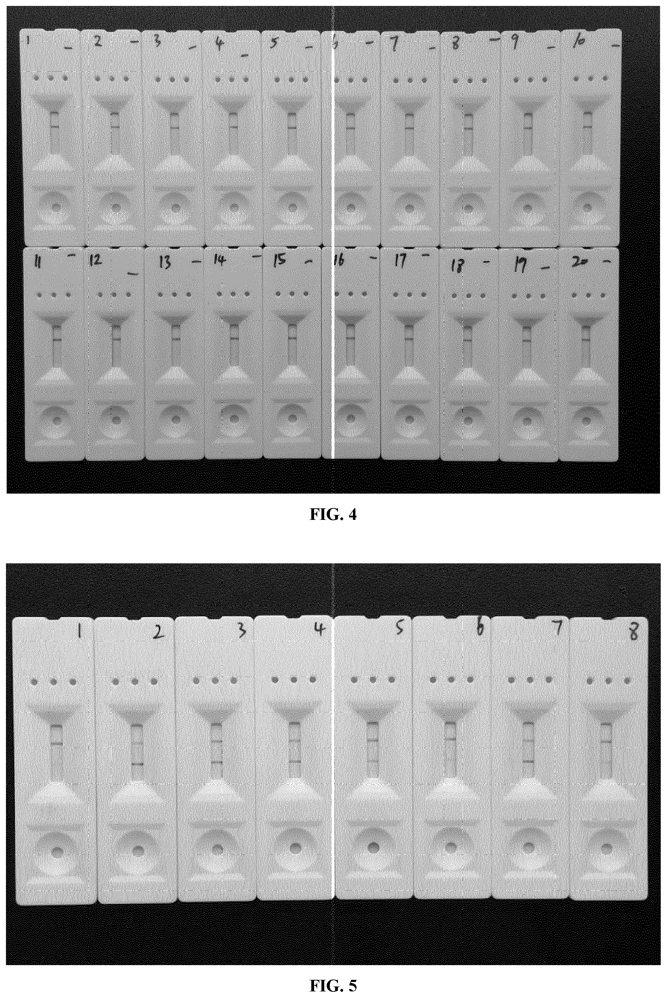

The test results in FIG. 4 and FIG. 5 show that the saliva samples of 20 non-pregnant women were tested negative, while the saliva samples of 8 pregnant women were tested positive; and after 5 min, the chromatography background was clean, the lines were clear, and the chromatography process was smooth. In summary, the test strip of the present disclosure had a high detection accuracy reaching 100%.

The above descriptions are merely preferred implementations of the present disclosure. It should be noted that a person of ordinary skill in the art may further make several improvements and modifications without departing from the principle of the present disclosure.

## Claims

1. A test strip for detecting human chorionic gonadotropin (HCG) in saliva, comprising a sample pad, a conjugate pad, an absorbent pad, a coating membrane, and a substrate, wherein the sample pad, the conjugate pad, the absorbent pad, and the coating membrane are sequentially overlapped on the substrate;
the sample pad is a glass fiber membrane treated with a sample pad treatment solution; and
each 100 mL of the sample pad treatment solution comprises the following components:
1.0 g to 1.5 g of Tris, 0.3 g to 0.8 g of casein, 50 µL to 100 µL of Tween-20, 0.5 g to 1.5 g of NaCl, 0.15 g to 0.2 g of dodecyltrimethylammonium bromide (DTAB), 0.8 g to 1.2 g of 4-nonylphenyl-polyethylene glycol, 3.5 g to 4.0 g of borax, 0.5 g to 1 g of polyvinylpyrrolidone (PVP), 3 g to 5 g of dithiothreitol (DTT), and 0.8 g to 2 g of L-cysteine.

2. The test strip according to claim 1, wherein the sample pad treatment solution has a pH value of 8.0 to 8.5.

3. The test strip according to claim 1, wherein the coating membrane is a nitrocellulose membrane coated with a test line (T line) and a control line (C line);
wherein the T line is sprayed with 0.5 mg/mL to 1 mg/mL of a mouse anti-α-HCG monoclonal antibody dilution;
the C line is sprayed with 0.4 mg/mL to 0.6 mg/mL of a goat anti-mouse immunoglobulin G (IgG) polyclonal antibody dilution; and
the T line or the C line is sprayed at an amount of 0.5 µL/cm to 1.5 µL/cm.

4. The test strip according to claim 3, wherein the mouse anti-α-HCG monoclonal antibody dilution or the goat anti-mouse IgG polyclonal antibody dilution solution is prepared by coating the mouse anti-α-HCG monoclonal antibody or the goat anti-mouse IgG polyclonal antibody with a coating diluent; and the coating diluent is a PBS solution with 50 mg/mL to 150 mg/mL of trehalose.

5. The test strip according to claim 1, wherein the conjugate pad is a glass fiber membrane or a polyester fiber membrane treated with a conjugate pad treatment solution; and
the conjugate pad treatment solution comprises the following components: 1 g to 1.5 g of 3-(N-morpholino)-2-hydroxy-propanesulfonic acid, 0.2 g to 1 g of casein, 50 µL to 100 µL of Tween-20, and 75 mL to 85 mL of water.

6. The test strip according to claim 5, wherein a β-HCG latex microsphere is sprayed on the glass fiber membrane or the polyester fiber membrane at 1.5 µL/cm to 3 µL/cm;
wherein β-HCG latex microsphere is prepared by the following steps:
washing a red latex microsphere with a borax buffer to obtain a washed red latex microsphere;
mixing an anti-β-HCG antibody with the washed red latex microsphere, and activating a resulting mixture using 1-ethyl-3-(3-dimethylaminopropyl)carbodiimide hydrochloride (EDC·HCl) solution to obtain an anti-β-HCG antibody-modified red latex microsphere; and
mixing an ethanolamine solution with the anti-β-HCG antibody-modified red latex microsphere to allow blocking to obtain a blocked β-HCG red latex microsphere, conducting centrifugation and removing a resulting supernatant; ultrasonically mixing the blocked β-HCG red latex microsphere with a Tris buffer evenly, conducting centrifugation and removing a resulting supernatant; ultrasonically mixing a remaining product with the Tris buffer evenly, and conducting rotary blending at a room temperature for 4 h to 6 h to obtain the β-HCG latex microsphere.

7. A method for preparing the test strip according to any one of claims 1 to 6, comprising the following steps:
(1) preparing the sample pad, comprising:
allowing the glass fiber membrane to absorb the sample pad treatment solution evenly, and drying the glass fiber membrane to obtain the sample pad; wherein the sample pad treatment solution comprises the following components in each 100 mL of sample pad treatment solution:
1.0 g to 1.5 g of Tris, 0.3 g to 0.8 g of casein, 50 µL to 100 µL of Tween-20, 0.5 g to 1.5 g of NaCl, 0.15 g to 0.2 g of dodecyltrimethylammonium bromide (DTAB), 0.8 g to 1.2 g of 4-nonylphenyl-polyethylene glycol, 3.5 g to 4.0 g of borax, 0.5 g to 1 g of polyvinylpyrrolidone (PVP), 3 g to 5 g of dithiothreitol (DTT), and 0.8 g to 2 g of L-cysteine;
(2) preparing the conjugate pad, comprising:
allowing the glass fiber membrane or the polyester fiber membrane to absorb the conjugate pad treatment solution evenly, drying, and spraying the β-HCG latex microsphere on the glass fiber membrane or the polyester fiber membrane treated with the conjugate pad treatment solution at a spraying volume of 1.5 µL/cm to 3 µL/cm to obtain the conjugate pad;
wherein the β-HCG latex microsphere are prepared by the following steps:
washing a red latex microsphere with a borax buffer to obtain a washed red latex microsphere;
mixing an anti-β-HCG antibody with the washed red latex microsphere, and activating a resulting mixture using 1-ethyl-3-(3-dimethylaminopropyl)carbodiimide hydrochloride (EDC·HCl) solution to obtain an anti-β-HCG antibody-modified red latex microsphere; and
mixing an ethanolamine solution with the anti-β-HCG antibody-modified red latex microsphere to allow blocking to obtain a blocked β-HCG red latex microsphere, conducting centrifugation and removing a resulting supernatant; ultrasonically mixing the blocked β-HCG red latex microsphere with a Tris buffer evenly, conducting centrifugation and removing a resulting supernatant; ultrasonically mixing a remaining product with the Tris buffer evenly, and conducting rotary blending at a room temperature for 4 h to 6 h to obtain the β-HCG latex microsphere;
(3) preparing the coating membrane, comprising:
diluting a mouse anti-α-HCG monoclonal antibody and a goat anti-mouse IgG polyclonal antibody with the coating diluent to obtain the mouse anti-α-HCG monoclonal antibody dilution and the goat anti-mouse IgG polyclonal antibody dilution, respectively; spraying the mouse anti-α-HCG monoclonal antibody dilution on the T line of the nitrocellulose membrane, spraying the goat anti-mouse IgG polyclonal antibody dilution on the C line of the nitrocellulose membrane, and drying the nitrocellulose membrane to obtain the coating membrane; and
(4) preparing the test strip:
cutting the sample pad, the conjugate pad, and the absorbent pad separately, and pasting the sample pad, the conjugate pad, the coating membrane, and the absorbent pad on the substrate in sequence; wherein one end of the conjugate pad is arranged above the sample pad, the other end of the conjugate pad is arranged below one end of the coating membrane, the other end of the coating membrane is arranged below the absorbent pad, and the C line on the coating membrane is close to the absorbent pad.

8. The method according to claim 7, wherein the anti-β-HCG antibody, the washed red latex microsphere, and the EDC·HCl solution are added at 1 mg: 10 mg: 0.05 mL.

9. A kit for detecting early pregnancy, comprising the test strip according to any one of claims 1 to 6 and a sample diluent.

## Patentansprüche

1. Teststreifen zum Nachweis von menschlichem Choriongonadotropin (HCG) in Speichel, umfassend ein Probenpad, ein Konjugatpad, ein Absorberpad, eine Beschichtungsmembran und ein Substrat, wobei das Probenpad, das Konjugatpad, das Absorberpad und die Beschichtungsmembran nacheinander auf dem Substrat überlappt sind;
das Probenpad eine mit einer Probenpad-Behandlungslösung behandelte Glasfasermembran ist; und
jede 100 mL der Probenpad-Behandlungslösung die folgenden Komponenten umfasst:
1,0 g bis 1,5 g Tris, 0,3 g bis 0,8 g Casein, 50 µL bis 100 µL Tween-20, 0,5 g bis 1,5 g NaCl, 0,15 g bis 0,2 g Dodecyltrimethylammoniumbromid (DTAB), 0,8 g bis 1,2 g 4-Nonylphenyl-Polyethylenglykol, 3,5 g bis 4,0 g Borax, 0,5 g bis 1 g Polyvinylpyrrolidon (PVP), 3 g bis 5 g Dithiothreitol (DTT) und 0,8 g bis 2 g L-Cystein.

2. Teststreifen gemäß Anspruch 1, wobei die Probenpad-Behandlungslösung einen pH-Wert von 8,0 bis 8,5 aufweist.

3. Teststreifen gemäß Anspruch 1, wobei die Beschichtungsmembran eine mit einer Testlinie (T-Linie) und einer Kontrolllinie (C-Linie) beschichtete Nitrocellulosemembran ist;
wobei die T-Linie mit 0,5 mg/ml bis 1 mg/ml einer Maus-anti-α-HCGmonoklonalen Antikörperverdünnung besprüht ist;
die C-Linie mit 0,4 mg/ml bis 0,6 mg/ml einer Ziegen-anti-Maus-Immunglobulin G (IgG) polyklonalen Antikörperverdünnung besprüht ist; und
die T-Linie oder die C-Linie mit einer Menge von 0,5 µL/cm bis 1,5 µL/cm besprüht ist.

4. Teststreifen gemäß Anspruch 3, wobei die Maus-anti-α-HCG-monoklonale Antikörperverdünnungs- oder die Ziege-anti-Maus-IgG-polyklonale Antikörperverdünnungslösung durch Beschichten des Maus-anti-α-HCG-monoklonalen Antikörpers oder des Ziege-anti-Maus-IgG-polyklonalen Antikörpers mit einem Beschichtungsverdünnungsmittel hergestellt ist; und das Beschichtungsverdünnungsmittel eine PBS-Lösung mit 50 mg/mL bis 150 mg/mL Trehalose ist.

5. Teststreifen gemäß Anspruch 1, wobei das Konjugatpad eine Glasfasermembran oder eine Polyesterfasermembran behandelt mit einer Konjugatpad-Behandlungslösung ist; und
die Konjugatpad-Behandlungslösung die folgenden Komponenten umfasst: 1 g bis 1,5 g 3-(N-Morpholino)-2-hydroxypropansulfonsäure, 0,2 g bis 1 g Casein, 50 µL bis 100 µL Tween-20 und 75 mL bis 85 mL Wasser.

6. Teststreifen gemäß Anspruch 5, wobei eine β-HCG-Latex-Mikrokugel auf die Glasfasermembran oder die Polyesterfasermembran mit 1,5 µL/cm bis 3 µL/cm gesprüht ist;
wobei die β-HCG-Latex-Mikrokugel durch die folgenden Schritte hergestellt ist:
Waschen einer roten Latex-Mikrokugel mit einem Borax-Puffer, um eine gewaschene rote Latex-Mikrokugel zu erhalten;
Mischen eines Anti-β-HCG-Antikörpers mit der gewaschenen roten Latex-Mikrokugel und Aktivieren des resultierenden Gemischs unter Verwendung einer 1-Ethyl-3-(3-dimethylaminopropyl)carbodiimid-Hydrochlorid (EDC·HCl)-Lösung, um eine anti-β-HCG-Antikörper-modifizierte rote Latex-Mikrokugel zu erhalten; und
Mischen einer Ethanolaminlösung mit der anti-β-HCG-Antikörper-modifizierten roten Latex-Mikrokugel, um eine Blockierung zu ermöglichen, um eine blockierte β-HCG rote Latex-Mikrokugel zu erhalten, Durchführen einer Zentrifugation und Entfernen eines resultierenden Überstandes; gleichmäßiges Mischen mittels Ultraschall der blockierten β-HCG roten Latex-Mikrokugel mit einem Tris-Puffer, Durchführen einer Zentrifugation und Entfernen eines resultierenden Überstandes; gleichmäßiges Mischen mittels Ultraschall eines verbleibenden Produkts mit dem Tris-Puffer und Durchführen eines Vermischens mittels Rotation bei Raumtemperatur für 4 h bis 6 h, um die β-HCG-Latex-Mikrokugel zu erhalten.

7. Verfahren zum Herstellen des Teststreifens gemäß einem der Ansprüche 1 bis 6, umfassend die folgenden Schritte:
(1) Herstellen des Probenpads, umfassend:
Ermöglichen, dass die Glasfasermembran die Probenpad-Behandlungslösung gleichmäßig absorbiert, und Trocknen der Glasfasermembran, um das Probenpad zu erhalten; wobei die Probenpad-Behandlungslösung die folgenden Komponenten in jeden 100 ml der Probenpad-Behandlungslösung umfasst:
1,0 g bis 1,5 g Tris, 0,3 g bis 0,8 g Casein, 50 µL bis 100 µL Tween-20, 0,5 g bis 1,5 g NaCl, 0,15 g bis 0,2 g Dodecyltrimethylammoniumbromid (DTAB), 0,8 g bis 1,2 g 4-Nonylphenyl-Polyethylenglykol, 3,5 g bis 4,0 g Borax, 0,5 g bis 1 g Polyvinylpyrrolidon (PVP), 3 g bis 5 g Dithiothreitol (DTT), und 0,8 g bis 2 g L-Cystein;
(2) Herstellen des Konjugatpads, umfassend:
Ermöglichen, dass die Glasfasermembran oder die Polyesterfasermembran die Konjugatpad-Behandlungslösung gleichmäßig absorbiert, Trocknen und Sprühen der β-HCG-Latex-Mikrokugel auf die Glasfasermembran oder die Polyesterfasermembran behandelt mit der Konjugatpad-Behandlungslösung mit einem Sprühvolumen von 1,5 µL/cm bis 3 µL/cm, um das Konjugatpad zu erhalten;
wobei die β-HCG-Latex-Mikrokugel durch die folgenden Schritte hergestellt wird:
Waschen einer roten Latex-Mikrokugel mit einem Borax-Puffer, um eine gewaschene rote Latex-Mikrokugel zu erhalten;
Mischen eines Anti-β-HCG-Antikörpers mit der gewaschenen roten Latex-Mikrokugel und Aktivieren des resultierenden Gemischs unter Verwendung einer 1-Ethyl-3-(3-dimethylaminopropyl)carbodiimid-Hydrochlorid (EDC·HCl)-Lösung, um eine anti-β-HCG-Antikörper-modifizierte rote Latex-Mikrokugel zu erhalten; und
Mischen einer Ethanolaminlösung mit der anti-β-HCG-Antikörper-modifizierten roten Latex-Mikrokugel, um eine Blockierung zu ermöglichen, um eine blockierte rote β-HCG-Latex-Mikrokugel zu erhalten, Durchführen einer Zentrifugation und Entfernen eines resultierenden Überstandes; gleichmäßiges Mischen mittels Ultraschall der blockierten β-HCG roten Latex-Mikrokugel mit einem Tris-Puffer, Durchführen einer Zentrifugation und Entfernen eines resultierenden Überstands; gleichmäßiges Mischen mittels Ultraschall eines verbleibenden Produkts mit dem Tris-Puffer und Durchführen eines Vermischens mittels Rotation bei Raumtemperatur für 4 h bis 6 h, um die β-HCG-Latex-Mikrokugel zu erhalten;
(3) Herstellen der Beschichtungsmembran, umfassend:
Verdünnen eines Maus-anti-α-HCG-monoklonalen Antikörpers und eines Ziegen-anti-Maus-IgG polyklonalen Antikörpers mit dem Beschichtungsverdünnungsmittel, um jeweils die Maus-anti-α-HCG-monoklonale Antikörperverdünnungslösung und die Ziege-anti-Maus-IgG-polyklonale Antikörperverdünnungslösung zu erhalten; Sprühen der Maus-anti-α-HCG-monoklonale Antikörperverdünnungslösung auf die T-Linie der Nitrocellulosemembran, Sprühen der Ziege-anti-Maus-IgG-polyklonale Antikörperverdünnungslösung auf die C-Linie der Nitrocellulosemembran und Trocknen der Nitrocellulosemembran, um die Beschichtungsmembran zu erhalten; und
(4) Herstellen des Teststreifens:
Schneiden des Probenpads, des Konjugatpads und des Absorperpads getrennt und Aufkleben des Probenpads, des Konjugatpads, der Beschichtungsmembran und des Absorberpads auf das Substrat in Folge; wobei ein Ende des Konjugatpads oberhalb des Probenpads angeordnet ist, das andere Ende des Konjugatpads unterhalb eines Endes der Beschichtungsmembran angeordnet ist, das andere Ende der Beschichtungsmembran unterhalb des Absorberpads angeordnet ist, und die C-Linie auf der Beschichtungsmembran nahe dem Absorberpad vorliegt.

8. Verfahren gemäß Anspruch 7, wobei der Anti-β-HCG-Antikörper, die gewaschene rote Latex-Mikrokugel und die EDC·HCl-Lösung zu 1 mg : 10 mg : 0,05 mL zugegeben werden.

9. Kit zum Detektieren einer Frühschwangerschaft, umfassend den Teststreifen gemäß einem der Ansprüche 1 bis 6 und ein Probenverdünnungsmittel.

## Revendications

1. Une bandelette réactive pour détecter la gonadotrophine chorionique humaine (HCG) dans la salive, comprenant un tampon échantillon, un tampon conjugué, un tampon absorbant, une membrane d'enrobage et un substrat, dans lequel le tampon échantillon, le tampon conjugué, le tampon absorbant et la membrane d'enrobage sont superposés séquentiellement sur le substrat ;
le tampon échantillon est une membrane de fibre de verre traitée avec une solution de traitement du tampon échantillon ; et
chacun des 100 mL de la solution de traitement du tampon échantillon comprend les composants suivants :
1,0 g à 1,5 g de Tris, 0,3 g à 0,8 g de caséine, 50 µL à 100 µL de Tween-20, 0,5 g à 1,5 g de NaCl, 0,15 g à 0,2 g de bromure de dodécyltriméthylammonium (DTAB), 0,8 g à 1,2 g de 4-nonylphényl-polyéthylène glycol, 3,5 g à 4,0 g de borax, 0,5 g à 1 g de polyvinylpyrrolidone (PVP), 3 g à 5 g de dithiothréitol (DTT), et 0,8 g à 2 g de L-cystéine.

2. La bandelette réactive selon la revendication 1, dans laquelle la solution de traitement du tampon échantillon a un pH de 8,0 à 8,5.

3. La bandelette réactive selon la revendication 1, dans laquelle la membrane d'enrobage est une membrane de nitrocellulose enrobée d'une ligne de test (ligne T) et d'une ligne de contrôle (ligne C) ;
dans lequel la ligne T est pulvérisée avec 0,5 mg/mL à 1 mg/mL d'une dilution d'anticorps monoclonal anti-α-HCG de souris ;
la ligne C est pulvérisée avec 0,4 mg/mL à 0,6 mg/mL d'une dilution d'anticorps polyclonal d'immunoglobuline G (IgG) de chèvre anti-souris ; et
la ligne T ou la ligne C est pulvérisée à une quantité de 0,5 µL/cm à 1,5 µL/cm.

4. La bandelette réactive selon la revendication 3, dans laquelle la solution de dilution d'anticorps monoclonal anti-α-HCG de souris ou la solution de dilution d'anticorps polyclonal IgG de chèvre anti-souris est préparée en enrobant l'anticorps monoclonal anti-α-HCG de souris ou l'anticorps polyclonal IgG de chèvre anti-souris avec un diluant d'enrobage ; et le diluant d'enrobage est une solution de PBS avec 50 mg/mL à 150 mg/mL de tréhalose.

5. La bandelette réactive selon la revendication 1, dans laquelle le tampon conjugué est une membrane de fibre de verre ou une membrane de fibre de polyester traitée avec une solution de traitement du tampon conjugué ; et
la solution de traitement du tampon conjugué comprend les composants suivants : 1 g à 1,5 g d'acide 3-(N-morpholino)-2-hydroxy-propanesulfonique, 0,2 g à 1 g de caséine, 50 µL à 100 µL de Tween-20, et 75 mL à 85 mL d'eau.

6. La bandelette réactive selon la revendication 5, dans laquelle une microsphère de latex β-HCG est pulvérisée sur la membrane de fibre de verre ou la membrane de fibre de polyester à 1,5 µL/cm à 3 µL/cm ;
dans laquelle la microsphère de latex β-HCG est préparée par les étapes suivantes :
laver une microsphère de latex rouge avec un buffer de borax pour obtenir une microsphère de latex rouge lavée ;
mélanger un anticorps anti-β-HCG à la microsphère de latex rouge lavée et activer le mélange résultant en utilisant une solution de chlorhydrate de 1-éthyl-3-(3-diméthylaminopropyl)carbodiimide (EDC·HCl) pour obtenir une microsphère de latex rouge modifiée par l'anticorps anti-β-HCG ; et
mélanger une solution d'éthanolamine avec la microsphère de latex rouge modifiée par l'anticorps anti-β-HCG pour permettre de bloquer pour obtenir une microsphère de latex rouge β-HCG bloquée, effectuer une centrifugation et enlever un surnageant résultant ; mélanger par ultrasons la microsphère de latex rouge β-HCG bloquée avec un buffer Tris de façon homogène, effectuer une centrifugation et enlever un surnageant résultant ; mélanger par ultrasons un produit restant avec le buffer Tris de façon homogène, et effectuer un mélange rotatif à une température ambiante pendant 4 h à 6 h pour obtenir la microsphère de latex β-HCG.

7. Un procédé de préparation de la bandelette réactive selon l'une quelconque des revendications 1 à 6, comprenant les étapes suivantes :
(1) préparer le tampon échantillon, comprenant :
permettre la membrane de fibre de verre d'absorber la solution de traitement du tampon échantillon de façon homogène, et sécher la membrane de fibre de verre pour obtenir le tampon échantillon ; dans lequel la solution de traitement du tampon échantillon comprend les composants suivants dans chacun des 100 mL de la solution de traitement du tampon échantillon :
1,0 g à 1,5 g de Tris, 0,3 g à 0,8 g de caséine, 50 µLà 100 µL de Tween-20, 0,5 g à 1,5 g de NaCl, 0,15 g à 0,2 g de bromure de dodécyltriméthylammonium (DTAB), 0,8 g à 1,2 g de 4-nonylphényl-polyéthylène glycol, 3,5 g à 4,0 g de borax, 0,5 g à 1 g de polyvinylpyrrolidone (PVP), 3 g à 5 g de dithiothréitol (DTT), et 0,8 g à 2 g de L-cystéine ;
(2) préparer le tampon conjugué, comprenant :
permettre la membrane de fibre de verre ou la membrane de fibre de polyester d'absorber la solution de traitement du tampon conjugué de façon homogène, sécher, et pulvériser la microsphère de latex β-HCG sur la membrane de fibre de verre ou la membrane de fibre de polyester traitée avec la solution de traitement du tampon conjugué à un volume de pulvérisation de 1,5 µL/cm à 3 µL/cm pour obtenir le tampon conjugué ;
dans lequel la microsphère de latex β-HCG est préparée selon les étapes suivantes :
laver une microsphère de latex rouge avec un buffer de borax pour obtenir une microsphère de latex rouge lavée ;
mélanger un anticorps anti-β-HCG à la microsphère de latex rouge lavée et activer le mélange résultant en utilisant une solution de chlorhydrate de 1-éthyl-3-(3-diméthylaminopropyl)carbodiimide (EDC·HCl) pour obtenir une microsphère de latex rouge modifiée par l'anticorps anti-β-HCG ; et
mélanger une solution d'éthanolamine avec la microsphère de latex rouge modifiée par l'anticorps anti-β-HCG pour permettre de bloquer pour obtenir une microsphère de latex rouge β-HCG bloquée, effectuer une centrifugation et enlever un surnageant résultant ; mélanger par ultrasons la microsphère de latex rouge β-HCG bloquée avec un buffer Tris de façon homogène, effectuer une centrifugation et enlever un surnageant résultant ; mélanger par ultrasons un produit restant avec le buffer Tris de façon homogène, et effectuer un mélange rotatif à une température ambiante pendant 4 h à 6 h pour obtenir la microsphère de latex β-HCG ;
(3) préparer la membrane d'enrobage, comprenant :
diluer un anticorps monoclonal anti-α-HCG de souris et un anticorps polyclonal IgG de chèvre anti-souris avec le diluant d'enrobage pour obtenir la dilution d'anticorps monoclonal anti-α-HCG de souris et la dilution d'anticorps polyclonal IgG de chèvre anti-souris, respectivement ; pulvériser la dilution d'anticorps monoclonal anti-α-HCG de souris sur la ligne T de la membrane de nitrocellulose, pulvériser la dilution d'anticorps polyclonal IgG de chèvre anti-souris sur la ligne C de la membrane de nitrocellulose, et sécher la membrane de nitrocellulose pour obtenir la membrane d'enrobage ; et
(4) préparer la bandelette réactive :
couper le tampon échantillon, le tampon conjugué et le tampon absorbant séparément, et coller le tampon échantillon, le tampon conjugué, la membrane d'enrobage et le tampon absorbant sur le substrat dans l'ordre ; dans lequel une extrémité du tampon conjugué est agencée au-dessus du tampon échantillon, l'autre extrémité du tampon conjugué est agencée sous une extrémité de la membrane d'enrobage, l'autre extrémité de la membrane d'enrobage est agencée sous le tampon absorbant, et la ligne C sur la membrane d'enrobage est proche du tampon absorbant.

8. Le procédé selon la revendication 7, dans lequel l'anticorps anti-β-HCG, la microsphère de latex rouge lavée et la solution d'EDC·HCl sont ajoutés à 1 mg : 10 mg : 0,05 mL.

9. Un kit de détection de grossesse précoce, comprenant la bandelette réactive selon l'une quelconque des revendications 1 à 6 et un diluant d'échantillon.
